(19) Europäisches Patentamt
European Patent Office
Office européen des brevets

(11) Publication number: **0 234 799**
**A2**

# (12) EUROPEAN PATENT APPLICATION

(21) Application number: 87301147.2

(22) Date of filing: 10.02.87

(51) Int. Cl.³: C 12 P 21/02
C 12 Q 1/68, G 01 N 33/68
G 01 N 33/58

(30) Priority: 11.02.86 DD 298835

(43) Date of publication of application: 02.09.87 Bulletin 87/36

(84) Designated Contracting States: BE CH DE FR GB IT LI LU NL SE

(71) Applicant: AMERSHAM INTERNATIONAL plc
Amersham Place
Little Chalfont Buckinghamshire HP7 9NA(GB)

(71) Applicant: Akademie der Wissenschaften der DDR
Otto-Nuschke-Strasse 22/23
DDR-1086 Berlin(DD)

(72) Inventor: Kurzchalia, Teymuras V.
Seefelder Strasse 36
DD-1092 Berlin(DD)

(72) Inventor: Wiedmann, Martin
Karlmarx Strasse 53
DD-1542 Falkensee(DD)

(72) Inventor: Bielka, Heinz
Robert Roessle Strasse 3
DD-1115 Berlin(DD)

(72) Inventor: Rapoport, Tom A.
Laudaer Strasse 38
DD-1100 Berlin(DD)

(74) Representative: Pennant, Pyers et al,
Stevens, Hewlett & Perkins 5 Quality Court Chancery Lane
London, WC2A 1HZ(GB)

## (54) Method of protein detection or isolation.

(57) A method for protein detection or isolation comprises the steps of: loading t-RNA with an amino acid such as lysine using an aminoacyl-t-RNA synthetase enzyme to form aminoacyl-t-RNA; chemically modifying the amino acid of the aminoacyl-t-RNA to form a modified aminoacyl-t-RNA; adding the modified aminoacyl-t-RNA to a cell-free protein synthesizing system to form a synthesized protein containing the modified amino acid; and detecting or isolating the synthesized protein or using the synthesized protein for the isolation and/or detection of another protein. The modified amino acid carries a modifying group joined to the amino acid by a linking group which may be cleavable. The modifying group may, for example, be a photoactivatable group, a radioactively labelled group, a directly detectable non-radioactive group, or a non-radioactive group having affinity for a specific protein, such as biotin or dinitrophenol.

EP 0 234 799 A2

0234799

# METHOD FOR PROTEIN DETECTION OR ISOLATION

The invention concerns a method for protein detection and isolation. There is described a procedure for the labelling and subsequent use of proteins prepared by means of a modified aminoacyl-t-RNA. A wide range of useful groups can be incorporated in the protein:

1) for non-radioactive detection;
2) for radioactive detection;
3) for cross-linking proteins to their interacting neighbours. The cross-links may be cleavable or non-cleavable.
4) for isolation/purification.

The invention is expected to be of interest in the production of bio- and radio-chemicals, particularly for molecular biological and biochemical laboratories.

## Background

In cases of structural and functional examination of biological systems it is often necessary to demonstrate that two molecules interact directly with each other or to find out which is the reaction partner of a given molecule.

Especially useful for this has proved to be photoaffinity labelling. The molecule to be examined is labelled with a chemical group which becomes chemically active through stimulation by light of a defined wave length, and thereupon reacts with another molecule, e.g. a protein, in its immediate environment. If the group chosen is a small one, a bond is achieved only with molecules which have formed a specific interaction with the molecule to be examined. Thus for instance proteins can be cross-linked, and it can be established which of them interacts specifically with one another. (Methods in Enzymology, 1976, 46).

In known procedures, the protein molecule to be

examined is modified after synthesis an exception being the use of iodotyrosine referenced below. This assumes most of the time that the protein is present in pure form. Loss of biological activity as a result of modification is quite often observed. It is not possible with the known procedures to examine interaction of a protein molecule during the course of its synthesis.

The bio-synthesis of proteins is usually observed via the incorporation of radioactively labelled amino acids. For this it is considerably easier to observe 35-S labelled proteins than 3-H labelled ones. However, it is a disadvantage that in most proteins the sulphur-containing amino acids are present only in small proportions. For many applications it is of great importance to increase the number of amino acids which are 35-S labelled. Further, it is desirable to introduce isotopes with harder, e.g. gamma-, radiation. With this the detection limit and detection time of newly synthesized proteins could be considerably reduced.

A method of iodinating in vitro synthesized proteins using iodotyrosyl-t-RNA has been described (N.H.Sherberg, J. Biol. Chem., 253(6) pages 1773 to 1779, 1978 and US Patents 4260737 and 4383033). Although 125-I and 131-I are readily detectable radionuclides, the method suffers from the disadvantage that tyrosine also occurs infrequently in proteins.

The use of non-radioactive labels has achieved considerable importance in many applications, e.g. in immunological techniques and nucleic acid labelling. Non-radioactive labelling of cell-free synthesized proteins has hitherto not been possible.

The bio-synthesis of proteins in cell-free translation systems supplies only very small quantities

which are then contained in a large surplus of existing mixtures of other proteins. Isolation of a newly synthesized protein in chemically pure form has hitherto not been possible. Chemical, and not just radiochemical, purity of the translation products would be of great importance for many areas of application.

Description of the Invention

A method of incorporating N-acetyl-lysine in bio-synthesized proteins by the use of N-acetyl-lysyl-t-RNA has been described (A.E.Johnson et al., Biochemistry 15(3) pages 569 to 575, 1976). However, the specific incorporation of photoaffinity reagents and biotin or other haptens offer distinct advantages not available with existing technology.

In one aspect the invention provides a method for protein detection or isolation comprising the steps of

- loading t-RNA with an amino acid using an aminoacyl-t-RNA synthetase enzyme to form aminoacyl-t-RNA
- chemically modifying the amino acid of the aminoacyl-t-RNA to form a modified aminoacyl-t-RNA,
- adding the modified aminoacyl-t-RNA to a cell-free protein synthesizing system to form a synthesized protein containing the modified amino acid,
- detection or isolation of the synthesized protein or using the synthesized protein for the isolation and/or detection of another protein.

The incorporation of modified amino acids into protein molecules is normally limited by the selectivity of the aminoacyl-t-RNA synthetase enzymes; normally only the twenty naturally occurring amino acids are accepted. By means of the invention, this selectivity barrier is avoided, since modification of the amino acid only takes place when it is already attached the t-RNA. A wide range of modifications

of the aminoacyl-t-RNA are accepted by the protein synthesizing machinery.

Suitable amino acids are those which carry a functional group in their side chain. Such groups can for instance be $NH_2$, OH, SH, COOH or imidazole. Especially useful is the amino acid lysine, whose epsilon amino group is readily accessible for modification. Other useful amino acids include glutamate, aspartate, cysteine, serine and threonine. A convenient way of attaching the modifying group to the functional group of the amino acid is by the use of an N-hydroxysuccinimide ester. Another similar coupling group, having the advantage of enhanced water solubility is the N-sulphosuccinimidyl group derived from N-hydroxysulphosuccinimide.

Coupling groups based on maleimide are suitable for reaction with thiol groups. Many other coupling groups are reported in the literature. The Radiochemical Centre technical Bulletin 79/6 gives a non-exhaustive list, with literature references of labelling reagents and suitable coupling groups.

The amino acid may be modified through attachment of a modifying group to the functional group of the amino acid, by means of a linkage which may be cleavable. The linkage may be a chain of from 1 to 20 methylene groups, which may contain a cleavable group such as disulphide -S-S-, or an azo linkage -N=N-, or a photocleavable group of known type.

According to an aspect of the invention, the amino acid of the aminoacyl-t-RNA may be modified through attachment thereto of a photoactivatable group. For example, lysyl-t-RNA may be treated with an N-hydroxysuccinimide ester of p-azido-benzoic acid or a derivative thereof or trifluoromethyl-diazyryl-benzoic acid, so that the photoactivatable group is attached to the epsilon-amino group. These photoactivatable groups can be activated by light of wave length 313 nm

or 320 nm. More generally, a photoaffinity labelling reagent may have the formula 1

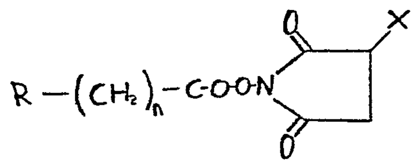

where R = 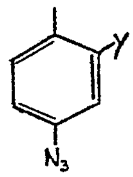 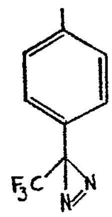 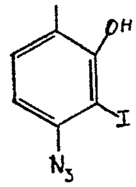

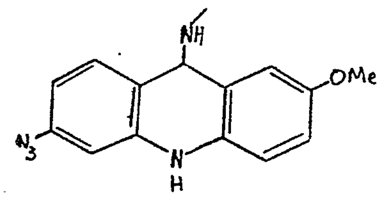

X = $SO_3^- Na^+$, H

Y = $NO_2$, H

n = 1 - 20

As noted above, the methylene chain may contain a cleavable group such as disulphide or azo, or a photocleavable group activated at a different light wave length from that used for the photoactivatable group. For this purpose the reagents SNAP and Denny-reagent are suitable.

After the modified amino acid has been introduced into a bio-synthesized protein, the photoactivatable group can be light activated. The reactive group thus generated binds to anything in its immediate neighbourhood. If the synthesized protein has interacted with another protein, the active group binds the two proteins together. Thereafter, known techniques can be used to detect or isolate the other

protein.

The method here described for labelling aminoacyl-t-RNA permits the introduction of 125-I via the Bolton and Hunter reagent. This can be effected either on all lysine molecules (or other amino acids having primary amino groups) or on specific lysyl molecules if individual lysine t-RNA species are utilised in the modification reaction.

The invention also permits the introduction of 35-S by the use of t-butoxycarbonyl-L-35S-methionine N-hydroxysuccinimidyl ester (SLR) to label lysine t-RNA. The use of this reagent offers the advantage over the use of 35S-methionine of increased specific activity of the proteins and therefore shorter autoradiography times.

It will be apparent to one skilled in the art that this principle can be extended to other nuclides such as 32-P in the phosphorylation of seryl-t-RNA. In addition, metal ions (e.g. Tc-99m) may be introduced via chelates.

After incorporation of the radioactive-modified amino acid in cell free-biosynthesized proteins and separation by known methods, detection can be achieved by means of autoradiography. In the case of 125-I, drying of the polyacrylamide gel is not necessary.

The use of non-radioactive labelling has specific advantages in terms of the stability of the labelled compounds and has received considerable attention, for example, with immunological techniques and nucleic acid labelling with biotin. Examples of non-radioactive chemical groups having affinity for specific proteins that may be attached to aminoacyl-t-RNA such as lysyl-t-RNA are: biotin, iminobiotin (with or without spacer arms), chromophores, luminescent or fluorescent groups such as fluorescein and texas red, dinitrophenol and other haptens. The specific protein concerned may

be either an antibody to the chemical group or, in the case of biotin, may be avidin or streptavidin.

A general procedure for the use of such reagents is as follows. First the chemical group having an affinity for a specific protein is coupled to the epsilon amino group of lysyl-t-RNA (or to a functional group on another aminoacyl-t-RNA). After incorporation of the modified amino acid into the synthesized protein, the protein can be identified by affinity to antibodies or other molecules which specifically bind to the chemical group. Identification can be carried out in accordance with the "Western-Blot" procedure with a radioactively labelled protein partner or with an enzyme-coupled protein partner.

The synthesis of proteins in cell-free translation systems provide very small quantities which are mixed with other proteins. The use of biotin or other chemical groups with affinity for specific proteins to label aminoacyl-t-RNA provides a method of purification of newly synthesized proteins. The detectable group may be linked to the aminoacyl-t-RNA by a cleavable or non-cleavable spacer arm. After synthesis of the protein, the reaction mixture may be treated with an immobilised affinity reagent, to which the synthesized protein becomes bound, the solid being recovered from the remainder of the reaction medium. If a cleavable linkage has been used, it may be cleaved at this stage, permitting recovery of the synthesized protein in pure form.

By way of example, using a mixture of a cleavable photoaffinity labelled lysyl-t-RNA and a biotinylated lysyl-t-RNA in the same translation experiment, it is possible to isolate protein molecules which have a specific interaction with the newly synthesized protein. Purification of such molecules can be accomplished by making use of the streptavidin system

as previously described.

It is possible to use either purified t-RNA species or cloned t-RNA genes in the method of this invention. This allows specific subsets of (for example) lysine residues in a protein to be labelled. This permits modifications to be directed at particular sites within the protein (for example labelling of lysine in the signal sequence, or avoidance of modification at an active site).

In another aspect, the invention provides as a novel material, aminoacyl-t-RNA in which the aminoacyl group carries a chemical group having affinity for a specific protein. As previously noted, the chemical group may be derived from biotin, iminobiotin, dinitrophenol or a fluorescent group or some other hapten, and may be linked to the aminoacyl group by means of a linkage which is cleavable.

In yet another aspect, the invention provides aminoacyl-t-RNA in which the aminoacyl group is derived from a naturally occurring amino acid other than tyrosine and carries a radioactively labelled group.

The invention offers the following advantages:

- Incorporation of chemical groups which can be photoactivated in proteins during their synthesis. This permits post- and co-translational interactions with other molecules.
- When using labelled amino acids which are normally placed on the surface of a folded protein molecule, the danger of disturbance of the biological function by the modification reaction is reduced.
- The detection limit and detection time of cell-free biosynthesized proteins can be considerably reduced.
- The use of radioactive labelling of newly

synthesized proteins can be avoided by the use of chemical groups which have an affinity for specific proteins.

- The method may be employed for non-radioactively labelled cell free synthesized products which have increased stability over the radioactively labelled versions, and which permit additional applications.
- Cell free synthesized proteins can be isolated by this method in chemically pure form, which was not possible previously.

The invention is illustrated by the following examples.

Example 1

Co-translational incorporation of lysine, which is labelled with a photoactivatable group in the E-position, in preprolactin

Total yeast t-RNA was labelled with $^3$H-lysine using a mixture of aminoacyl-t-RNA synthetases from rat livers and then purified on sephadex G25. In subdued light, the labelled t-RNA (0.18 mole; 300 $OD_{260}$ units) was dissolved in 10ul of ice cold double distilled water. To this was added 10ul of $KH_2PO_4$ (0.2M; pH 7.0) and 10ul of 12mM ABA-NHS. The pH of the reaction was adjusted to 10.4 with 0.12M KOH and after 3 minutes the ABA-lysyl-t-RNA was ethanol precipitated. The ABA-lysyl-t-RNA was purified from unmodified lysyl-t-RNA on BD-cellulose.

The ABA-$^3$H-lysyl-t-RNA was then added to an in vitro translation system consisting of wheat germ programmed with preprolactin-mRNA. In this way, $^3$H-labelled preprolactin was synthesized. It was demonstrated by enzymatic hydrolysis of the protein and subsequent HPLC that the radioactivity stemmed from the incorporation of ABA-$^3$H-lysine.

The cross-linking of nascent preprolactin chains with an interactive protein via the photoactivatable group introduced during translation is illustrated by

the interaction of a signal peptide with the signal recognition particle (SRP).

The cell free translation of preprolactin-mRNA was synchronised by addition of 7mGp 1 minute after the start of translation in a wheat germ system. SRP (30 units) was added to the translation mixture containing 16pMol of ABA-lysyl-t-RNA and 88 uC; of $^{35}S$ methionine per 50ul. The duration of the translation reaction was 15 minutes. This was followed by the addition of 2 ul of 1mM methionine and the incubation continued for 5 minutes. The mixture was then purified on sepharose CL 6b, (1 x 20cm), which had been equilibrated with a buffer containing 50mM HEPES pH 7.5, 150mM potassium acetate 6mM magnesium acetate and 0.01% nikkol. Fractions which corresponded with the exclusion volume were collected and 200ul aliquots irradiated with light (313nm; $10^5$ erg/$mm^2$ for 2 minutes. Aliquots, which correspond with a volume of 50ul of the original translation mixture were precipitated with trichloroacetic acid and electrophoresed on a 10-20% SDS polyacrylamide-gel using a similar quantity of non-irradiated material as a control.

It was shown that a new band with a molecular weight of 60KD was produced after irradiation of the arrested translation complex. This product could be precipitated with antibodies directed against the 54KD-polypeptide of the SRP. Thus the 60KD product represents the cross-linking product between a protein component of the SRP and the arrested preprolactin fragment (approx 8KD). Since the only lysines in this fragment are situated just before the hydrophobic part of the signal sequence it can be assumed that the signal peptide receptor was identified.

Completely analogous results were achieved with

diacyl-lysyl-t-RNA.

Example 2

Incorporation of cleavable cross-linking groups in proteins during their syntheses.

50uCi (Cysteamine $^{35}S$)-N-succinimidyl-3-(2-nitro-4 azidophenyl)-2-amino ethyldithio) propionate were evaporated to dryness, dissolved in 0.18ml DMSO and frozen. 90ul yeast t-RNA loaded with cold lysine was added to the solution (110 $OD_{260}$/ml). The reaction was initiated by addition of 30ul 0.5M CAPS. The incubation period was 15 minutes.

Following incorporation of the modified lysine in the protein chain, cross-linking with neighbouring molecules was achieved by irradiation with light (see Example 1) cross-linked protein molecules were cleaved using 50mM DTT.

Alternatively the Denny reagent (N-(4-(p-azido-m$^{125}$I-iodophenylazo)benzoyl)-3-aminopropyl-N-hydroxysuccinimide ester was used (1mCi). The cleavage was carried out with 20mM dithionite.

Subsequent steps were carried out as in Example 1.

Example 3

For the incorporation of 125-I in proteins, the E-amino group of the lysine was modified using the Bolton-Hunter reagent (N-succinimidyl 3-(4-hydroxyl-5 125-I iodophenyl) propionate). Total t-RNA was loaded with cold lysine (see Example 1). 12ul of the solution (110 $OD_{260}$/ml) (Approx. 0.8 nMole lysine) was added to 24ul frozen DMSO and the mixture was thawed on ice. 5mCi of the Bolton and Hunter reagent were dried down in the reaction vessel and 27ul of the reaction mixture was added. The reaction was started by the addition of 3ul of 0.5M CAPS buffer pH 10.4 followed by incubation on ice for 15-20 minutes. All further steps were analogous to those with ABA-lysyl-t-RNA (Example 1).

The incorporation of $^{35}S$ in protein was carried out via the $^{35}S$ sulphur labelling reagent (SLR) (t-butoxycarbonyl-L-$^{35}S$ methionine N-hydroxysuccinimidyl ester) (EPA 181061). The procedure was analogous to the one with the Bolton-Hunter reagent except that 2mCi of the SLR (800 Ci/mMol) were applied.

Following SDS polyacrylamide gel-electrophoreses proteins were visualised by autoradiography. Drying of gels was not required with iodine.

## Example 4

Incorporation of biotin in newly synthesized proteins.

0.3ml (110 $OD_{260}$/ml) yeast t-RNA was added to a 0.6ml frozen solution of biotinyl-succinimidyl ester in DMSO. After thawing the reaction was started with 0.1ml 0.5M CAPS-buffer. All further steps took place analogously to the procedure with ABA-lysine-t-RNA (Example 1).

The demonstration of the incorporation of the biotin group into the newly synthesized proteins took place via SDS-polyacrylamide-gel-electrophoresis, "Western Blotting" and detection via streptavidin and biotinylated polyalkaline phosphatase.

CLAIMS

1. A method for protein detection or isolation comprising the steps of:-

- loading t-RNA with an amino acid using an aminoacyl t-RNA synthetase enzyme to form aminoacyl-t-RNA
- chemically modifying the amino acid of the aminoacyl-t-RNA to form a modified aminoacyl-t-RNA,
- adding the modified aminoacyl-t-RNA to a cell-free protein synthesizing system to form a synthesized protein containing the modified amino acid
- detection or isolation of the synthesized protein or using the synthesized protein for the isolation and/or detection of another protein.

2. A method as claimed in claim 1, wherein the amino acid is selected from lysine, glutamate, aspartate, cysteine, serine and threonine.

3. A method as claimed in claim 1 or claim 2, wherein the amino acid carries a functional group selected from $-NH_2$, -OH, -SH, -COOH and imidazole on its side chain, through which it is chemically modified to form the modified aminoacyl-t-RNA.

4. A method as claimed in claim 3, wherein the amino acid is modified through attachment of a modifying group to the functional group by means of a linkage which is cleavable.

5. A method as claimed in any one of claims 1 to 4, wherein the amino acid is modified through attachment thereto of a photoactivatable group.

6. A method as claimed in claim 5, wherein a reagent selected from a hydroxysuccinimide ester of p-azidobenzoic acid, trifluoromethyl-diazyryl-benzoic acid, (cysteamine-35S)-(2-nitro-4-azidophenyl-2-amino ethyldithio)-propionic acid, and N-(4-(p-azido-m-125-I-iodophenylazo)-benzoyl)-3-amino-propionic acid is used to introduce the photoactivatable group.

7. A method as claimed in claim 5 or claim 6, comprising the additional step of protoactivating the photoactivatable group in the synthesized protein so as to cause the synthesized protein to bind another protein in its immediate neighbourhood.

8. A method as claimed in claim 7, wherein the other protein is thereby detected or isolated.

9. A method as claimed in any one of claims 1 to 3, wherein the amino acid is modified through attachment thereto of a radioactively labelled group.

10. A method as claimed in claim 9, wherein a reagent selected from t-butoxycarbonyl-L-35S-methionine N-hydroxysuccinimidyl ester and N-hydroxysuccinimidyl-3-(4-hydroxy-5-125I-iodophenyl)-propionate is used to introduce the radioactively labelled group.

11. A method as claimed in any one of claims 1 to 4, wherein the amino acid is modified through attachment thereto of a chemical group having affinity for a specific protein.

12. A method as claimed in claim 11, wherein a reagent selected from biotin, iminobiotin, dinitrophenol and chromophore, luminescent and fluorescent groups is used to introduce the chemical group having affinity for a specific protein.

13. A method as claimed in claim 11 or claim 12, comprising the additional step of using the specific protein to detect the synthesized protein.

14. A method as claimed in claim 11 or claim 12, comprising the additional step of using the specific protein to isolate or purify the synthesized protein.

15. Aminoacyl-t-RNA in which the aminoacyl group carries a chemical group having affinity for a specific protein.

16. Aminoacyl-t-RNA as claimed in claim 15, wherein the chemical group is derived from biotin, iminobiotin, dinitrophenol or a fluorescent group.

17. Aminoacyl-t-RNA as claimed in claim 15 or claim 16, wherein the chemical group is linked to the aminoacyl group by means of a linkage which is cleavable.

18. Aminoacyl-t-RNA in which the aminoacyl group is derived from a naturally occurring amino acid other than tyrosine and carries a radioactively labelled group.